# FASCICULE DE BREVET EUROPEEN

(11) **EP 1 443 871 B1**
(45) Date de publication et mention de la délivrance du brevet: **06.05.2009**
(21) Numéro de dépôt: 02790531.4
(22) Date de dépôt: 22.10.2002
(51) Int. Cl.: A61F 2/00, A61B 17/04

(54) **MINI SOUTENEMENT SOUS URETHRO-CERVICAL**
SUBURETHRALE/ZERVIKALE MINISTÜTZE
MINI SUB-URETHRAL/CERVICAL SUPPORT

(30) Priorité: 22.10.2001 FR 0113585
(43) Date de publication de la demande: 11.08.2004
(73) Titulaire: Johnson & Johnson International, New Brunswick, NJ 08933 (US)
(72) Inventeur: ARNAUD, Axel, F-75015 Paris (FR); BOUFFIER, Bernard, F-25480 Ecole (FR)
(74) Mandataire: Callon de Lamarck, Jean-Robert
(86) Numéro de dépôt international: PCT/FR2002/003608
(87) Numéro de publication internationale: WO 2003/034939

(56) Documents cités:
- EP-A- 0 643 945
- EP-A- 0 684 012
- EP-A- 0 755 656
- WO-A-00/66029
- WO-A-00/74633
- US-A- 5 013 292
- US-A- 5 256 133
- US-A- 5 934 283

## Description

La présente invention concerne le domaine des traitements contre les incontinences urinaires.

L'incontinence urinaire chez la femme peut être due à un relâchement des tissus ou ligaments reliant la paroi vaginale au muscle pelvien et à l'os du pubis. Ces tissus ou ligaments constituent un ensemble musculo-ligamentaire et conjonctif qui permet le soutien de l'urètre et qui avec le sphincter de l'urètre participent activement au mécanisme de continence à l'effort.

Une solution connue pour éviter l'incontinence consiste à placer une bande de soutien en polypropylène sous l'urètre. Le document US 5 899 909 déposé par Medscand Medical AB (Suède) décrit un instrument chirurgical et un procédé permettant de traiter l'incontinence chez la femme sans nécessiter l'ouverture de l'abdomen. L'instrument comprend deux tiges métalliques courbes et une bande dont chaque extrémité est reliée à l'une des tiges. Les tiges sont munies de poignées amovibles qui permettent au chirurgien de les manipuler. Le chirurgien introduit les tiges l'une après l'autre par le vagin de part et d'autre de l'urètre. Chaque aiguille est enfoncée à travers la paroi vaginale et le plexus veineux et est guidée le long de l'os pubien pour traverser les muscles abdominaux. Le chirurgien extrait les aiguilles au travers de la paroi abdominale. La bande se met en place automatiquement en formant une boucle autour de l'urètre. Cette bande constitue une structure de renforcement et de soutien qui apporte un appui au sphincter. Elle reste en place dans le corps de la patiente du seul fait des forces de contact des tissus traversés sur la longueur de la bande.

Une telle méthode présente l'avantage d'être particulièrement peut invasive. C'est pourquoi elle connaît un grand succès.

Cependant, lors du passage des aiguilles, il existe un risque non négligeable que celles-ci traversent la paroi de la vessie. Il existe également des risques (plus minimes) que les aiguilles endommagent l'intestin ou un vaisseau sanguin.

un dispositif de soutènement selon le préambule de la revendication 1 est connu du document US-A-5013292.

Le but de la présente invention est de fournir un dispositif permettant de limiter ces risques. En effet, les principales complications des dispositifs prothétiques anciens sont dominées par les perforations de la vessie, de vaisseaux sanguins, voire de l'intestin.

A cet effet, l'invention propose un dispositif de soutènement sous uréthro-cervical conforme à la revendication 1.

Un tel dispositif permet avantageusement d'implanter, à l'aide des aiguilles, des éléments d'ancrage dans les tissus internes du corps de la patiente. Plus particulièrement, les éléments d'ancrage sont positionnés sur l'aponévrose pelvienne, ce qui présente l'avantage que les aiguilles ne nécessitent pas d'être enfoncées au-delà de l'os pubien et ne risquent donc pas d'endommager la vessie, les intestins ou les vaisseaux sanguins.

Un avantage de la présente invention est que l'opération d'implantation du soutènement est particulièrement simple et peu invasive. Elle ne nécessite pas de perforation des muscles abdominaux.

Un autre avantage de la présente invention est que l'opération d'implantation ne nécessite pas de procéder à une cystoscopie pour vérifier l'état de la vessie de la patiente, cette cystoscopie pouvant être source de risques infectieux.

Dans une mise en oeuvre de l'invention, le dispositif comprend deux bandelettes chacune ayant une extrémité fixée à l'un des éléments d'ancrage. Les bandelettes sont introduites et guidées par les aiguilles à travers la paroi du vagin et jusqu'à l'aponévrose pelvienne, chacune à leur tour. Elles sont ensuite reliées l'une à l'autre par des moyens de connexion pour former une boucle de soutènement sous l'urètre.

Cette caractéristique présente l'avantage que la longueur de la boucle de soutènement peut être ajustée par le chirurgien. Celui-ci peut donc régler précisément la pression exercée par le dispositif de soutènement sous l'urètre et l'adapter à la morphologie de la patiente.

Dans une mise en oeuvre particulièrement avantageuse de l'invention, l'aiguille est creuse et forme un canal débouchant à ses deux extrémités et l'extrémité de l'aiguille ne supportant pas l'élément d'ancrage est connectée à des moyens d'injection/aspiration par exemple pour introduire un produit anesthésiant dans le canal, ou insuffler un liquide facilitant le développement des éléments souples d'ancrage par dégagement des tissus environnants, ou encore pour aspirer une partie du fluide environnant la pointe de l'aiguille d'implantation afin de vérifier la zone de positionnement de celle-ci.

L'invention propose en outre un instrument chirurgical pour l'implantation d'un soutènement sous uréthro-cervical conforme à la revendication 11.

D'autres caractéristiques et avantages ressortiront encore de la description qui suit, laquelle est purement illustrative et non limitative et doit être lue au regard des figures annexées parmi lesquelles :
- la figure 1 est une représentation schématique du détail d'un instrument d'ancrage conforme à un mode de réalisation de l'invention,
- la figure 2 est une représentation schématique d'un exemple de soutènement sous uréthro-cervical conforme à l'invention,
- les figures 3 à 7 représentent schématiquement les différentes étapes d'un premier mode de mise en place d'un dispositif de soutènement,
- la figure 8 est une représentation schématique d'une pince permettant de régler avec précision la longueur de la boucle de soutènement,
- la figure 9 est une représentation schématique du détail d'un instrument d'ancrage conforme à un deuxième mode de réalisation de l'invention,
- la figure 10 représente un trocart tel qu'utilisé dans un deuxième mode de mise en place d'un dispositif de soutènement,
- la figure 11 est une représentation schématique d'une variante de pince permettant de positionner un élément de « clipsage » destiner à lier deux bandelettes pour former une boucle de soutènement,
- la figure 12 représente un exemple d'un tel élément de « clipsage »,
- la figure 13 représente une exemple d'embout destiné à la formation d'un élément d'ancrage,
- les figures 14 et 15 représentent schématiquement le positionnement d'une bandelette de soutènement dans l'embout de la figure 13 pour former un élément d'ancrage.

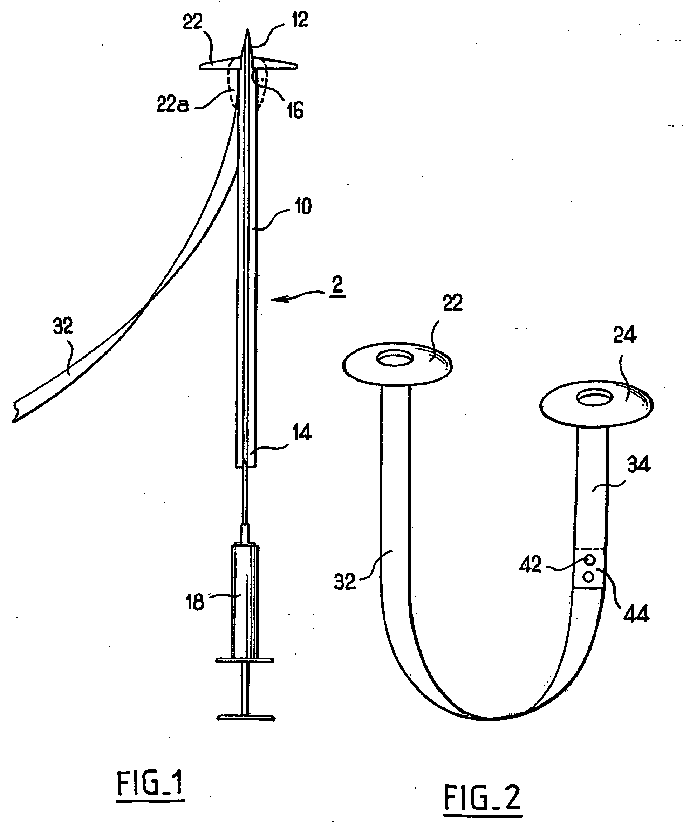

Selon la figure 1, le dispositif d'ancrage 2 comprend une aiguille 10 d'environ deux millimètres de diamètre dont l'extrémité 12 destinée à pénétrer à travers les tissus présente une forme générale conique. Cette aiguille 10 a été usinée à proximité de son extrémité 12 de manière à former une surface d'appui 16 en forme de couronne perpendiculaire à sa direction longitudinale. Un élément d'ancrage 22 a été installé emmanché sur l'extrémité 12 de l'aiguille 10 et en appui sur la surface 16. Cet élément d'ancrage 22 est constitué d'une rondelle souple d'environ 3 millimètres d'épaisseur et 10 millimètres de diamètre en silicone renforcé avec du polypropylène. La rondelle présente une forme de cône dont la concavité est dirigée vers l'extrémité 14 de manipulation de l'aiguille 10 opposée à la pointe 12. Cette caractéristique évite que la rondelle puisse être rabattue vers la pointe 12 de l'aiguille. En revanche, cette rondelle est assez souple pour être rabattue dans la position 22a (représentée en pointillés sur la figure 1) le long de l'aiguille 10 lorsque celle-ci est enfoncée à travers des tissus biologiques. Une extrémité d'une bandelette autostatique de polypropylène 32 est fixée sur l'élément d'ancrage 22, par exemple par collage ou tout autre moyen de fixation approprié.

La figure 2 représente un exemple de soutènement sous uréthro-cervical conforme à l'invention. Il comporte, deux éléments d'ancrages 22 et 24 sous forme de deux rondelles souples en silicone renforcée avec du polypropylène. Chaque rondelle 22 et 24 est reliée à une bandelette autostatique formée par un treillis de mèches de polypropylène et référencée respectivement 32 et 34. Chaque bandelette 32 et 34 comporte un élément de fixation 42, 44. Comme illustré sur la figure 2, les éléments de fixation 42 et 44 présentent des formes complémentaires et sont aptes à être engagés l'un dans l'autre par pression.

On va maintenant décrire différentes étapes d'un premier mode d'implantation du dispositif de soutènement sous uréthro-cervical dans le corps d'une patiente.

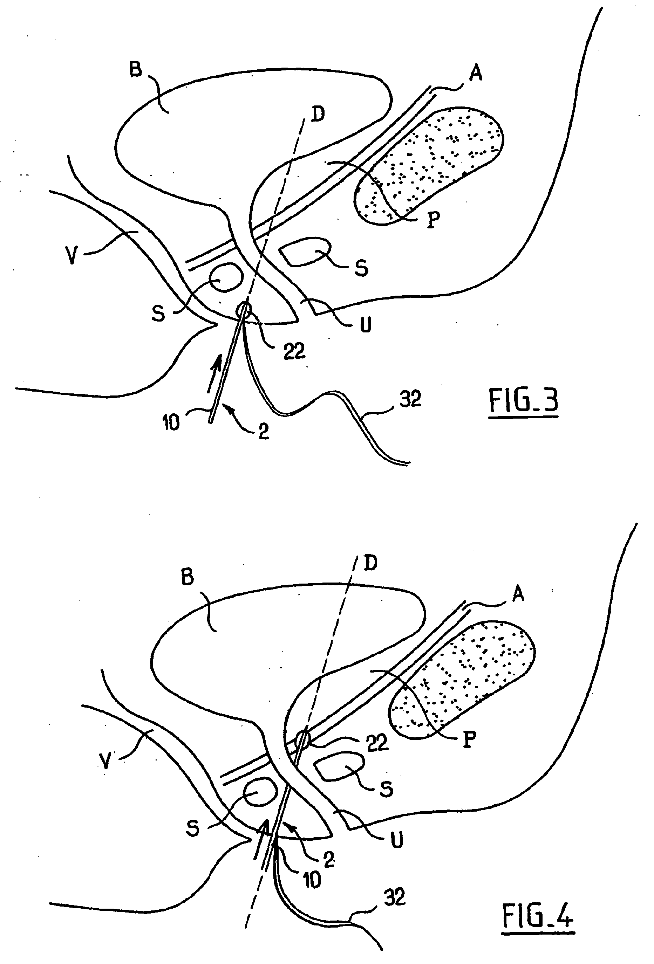

La figure 3 représente une première étape de l'implantation consistant à introduire un premier dispositif d'ancrage 2 supportant une bandelette 32. Durant cette étape, l'aiguille 10 est initialement introduite à travers la paroi du vagin V à proximité de l'urètre U. L'élément d'ancrage 22 est rabattu le long de l'aiguille. L'aiguille 10 est enfoncée selon la direction D entre les muscles S du sphincter et vers le plexus veineux P.

Sur la figure 4, on a représenté une seconde étape durant laquelle l'aiguille 10 traverse l'aponévrose pelvienne A, c'est à dire la membrane constituée de fibres conjonctives denses qui enveloppe les muscles du sphincter. A cet instant, le chirurgien peut sentir qu'il vient de traverser une partie de tissus moins tendre. Il cesse alors de pousser l'aiguille 10.

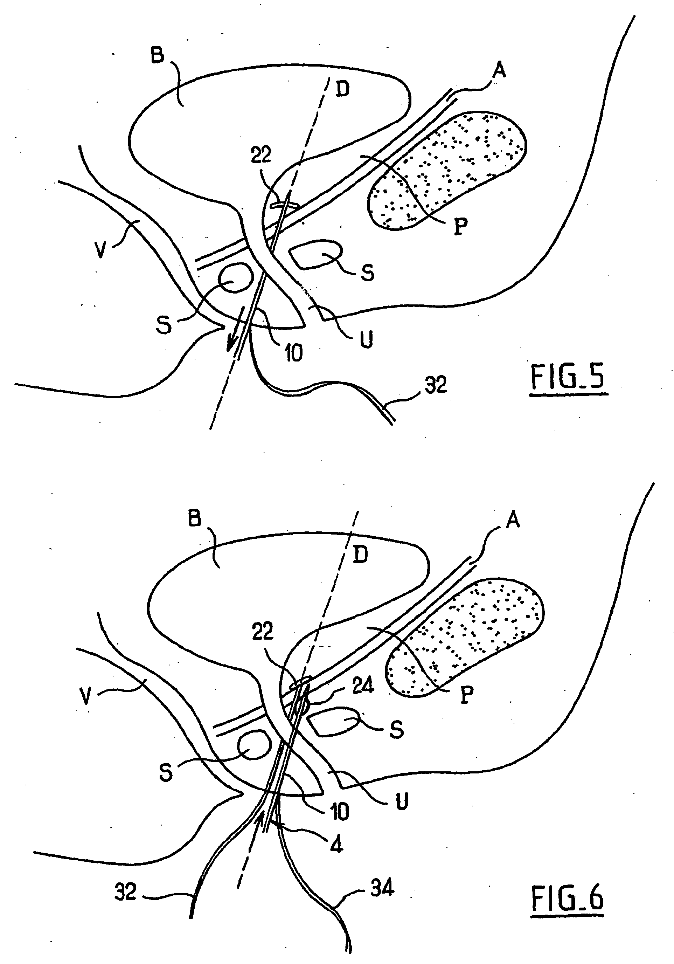

Comme illustré sur la figure 5, l'aiguille 10 est retirée dans la direction D, ce qui entraîne le déploiement de l'élément d'ancrage 22 dont les bords viennent en appui sur la surface de l'aponévrose pelvienne A. Lorsque le chirurgien retire l'aiguille 10, l'élément d'ancrage 22 se déploie et s'appuie sur la surface de l'aponévrose A, ce qui l'empêche d'être entraîné par l'aiguille 10. L'élément d'ancrage 22 se désolidarise alors de l'aiguille 10 et reste « ancré » sur l'aponévrose A. La bandelette 32 fixée par son extrémité à celui-ci est ainsi maintenue en place.

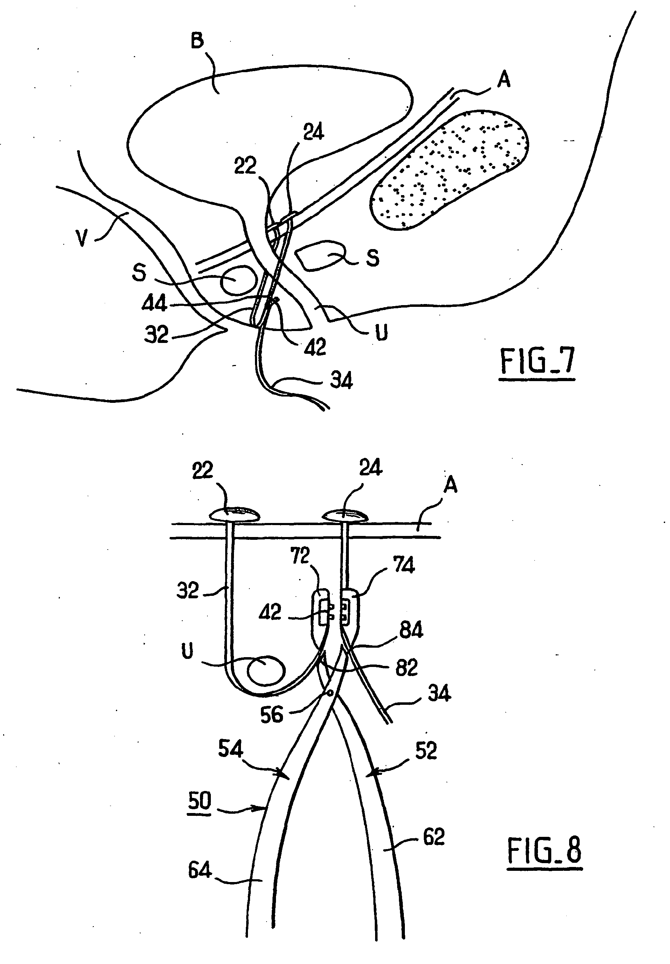

Comme illustré sur la figure 6, on recommence la même opération avec un deuxième dispositif d'ancrage 4 supportant une bandelette 34. Ce dispositif d'ancrage 4 est introduit à travers la paroi du vagin V dans la direction du plexus veineux P, mais de l'autre côté de l'urètre U par rapport à la première bande 32 déjà en place. Chaque partie de bande 32 et 34 ainsi positionnée passe de chaque côté de l'urètre U. Puis, ainsi qu'illustré sur la figure 7, le chirurgien relie les deux extrémités des bandelettes et règle la longueur de bandelette entre les deux dispositifs d'ancrage 22 et 24 et donc la pression exercée par celles-ci au niveau de l'urètre U. Les deux bandelettes 32 et 34 sont alors reliées l'une à l'autre par les moyens de clipsage 42 et 44 pour former une boucle de soutien sous l'urètre U.

Bien entendu, ces bandelettes 32 et 34 pourraient être reliées de manière différente. Elles peuvent être agrafées, suturées ou être accrochées par tout autre moyen adapté. L'urètre étant un organe fragile, l'accrochage se fera de préférence le plus loin possible de l'urètre U pour ne pas risquer de l'endommager. A cet effet, les deux bandelettes 32 et 34 sont coupées à des longueurs différentes:

Dans une variante de ce mode d'implantation du dispositif de soutènement, les bandelettes 32 et 34 sont entourées d'une gaine souple formée typiquement d'une feuille de matière plastique, qui facilite leur glissement durant les étapes d'introduction des aiguilles représentées aux figures 4 et 6. Ces gaines sont retirées par coulissement le long des bandelettes lorsque celles-ci sont en place dans le corps de la patiente.

Les gaines présentent l'avantage de faciliter l'introduction des bandelettes, celles-ci étant autostatiques. Les gaines présentent en outre l'avantage de protéger les bandelettes durant leur mise en place et d'éviter leur contamination par des bactéries des régions qu'elles traversent.

Sur la figure 8, on a représenté un exemple de pince permettant de régler facilement la longueur de la boucle constituée par les deux bandelettes 32 et 34. La pince 50 est constituée de deux bras 52 et 54 articulés au niveau d'un axe 56. Les bras 52 et 54 présentent respectivement deux extrémités 62, 72 et 64, 74. Les extrémités 62 et 64 formant levier permettent au chirurgien de serrer la pince et les extrémités 72 et 74 constituent les « mâchoires » de serrage. Chacune des extrémités 72 et 74 constituant les mâchoires présente une fente 82 ou 84 qui débouche latéralement et à travers laquelle peut venir coulisser l'une des bandelettes 32 et 34.

L'une des bandelettes 34 présente à une longueur prédéterminée un élément de fixation 42 venant être positionné sur l'une des mâchoires 72 de la pince. L'autre bandelette 34 passe à travers la fente de la mâchoire 74 opposée. Le chirurgien peut faire coulisser la bandelette 34 à travers la fente 84 de manière à régler la longueur de la boucle du soutènement passant sous l'urètre U. Lorsqu'il a trouvé la bonne longueur, il ferme la pince 50 de manière à serrer l'élément de fixation 42 de la bande 32 sur la bande 34 en une position adéquate.

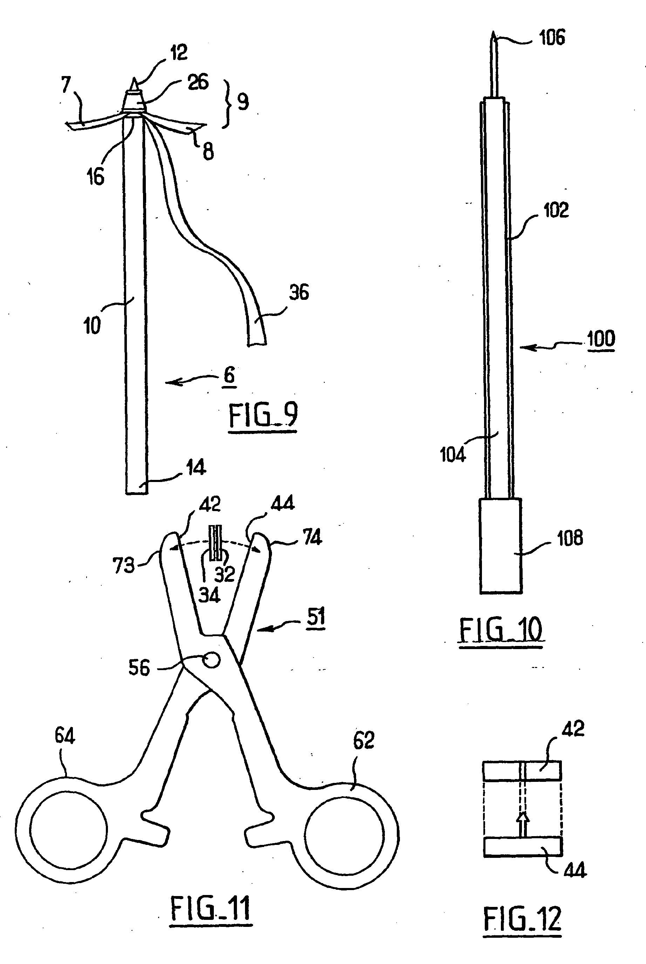

Une variante de pince a été représenté sur la figure 11. Cette pince 51 est constituée de deux bras 52 et 54 articulés au niveau d'un axe 56. Les bras 52 et 54 présentent respectivement deux extrémités 62, 72 et 64, 74. Les extrémités 72 et 74 constituant les mâchoires de serrage présentent des cavités de forme générale cylindrique adaptées pour recevoir des éléments de « clipsage » référencés 42 et 44. Lorsque le chirurgien souhaite accrocher les deux bandes 32 et 34 entre elles pour former la boucle de soutènement, il les superpose et positionne les mâchoires 72 et 74 de la pince 51 transversalement aux bandelettes 32 et 34, respectivement de part et d'autre de celles-ci.

La figure 12 représente plus précisément un exemple non-limitatif d'éléments de clipsage 42 et 44 du type bouton pression. L'élément 42 présente une forme générale de rondelle perforée tandis que l'élément 44 présente une forme de rondelle munie d'un pion. Le pion est apte à perforer les bandes 32 et 34 et à venir s'engager et se fixer dans l'orifice de l'élément 42. A cette fin, le pion présente une section droite de dimension inférieure à la perforation de la rondelle 42, sur l'essentiel de sa longueur, mais une tête évasée de dimension en section supérieure à cette perforation.

Dans une mise en oeuvre de l'invention illustrée à la figure 1, l'aiguille 10 est creuse et forme un conduit s'étendant entre ses extrémités 12 et 14. Une seringue 18 peut être connectée à l'embouchure du conduit située à l'extrémité 14 de l'aiguille 10. Cette seringue 18 peut être remplie d'un produit anesthésiant. Au cours de l'étape représentée à la figure 3 consistant à faire pénétrer l'aiguille 10 dans le corps de la patiente, le chirurgien pousse sur le piston de la seringue 18. Il injecte le produit anesthésiant via le conduit formé par l'aiguille 10 pour réaliser une anesthésie locale progressive à mesure de la pénétration de l'aiguille 10 à travers le corps de la patiente.

Cette mise en oeuvre de l'invention est particulièrement avantageuse car elle permet au chirurgien de réaliser une anesthésie extrêmement bien localisée du corps de la patiente. En outre, elle présente l'avantage de permettre au chirurgien de détecter l'atteinte éventuelle de la vessie B. A cet effet, le chirurgien peut tirer légèrement sur le piston de la seringue 18. Si le piston résiste, cela signifie que l'extrémité 12 de l'aiguille 10 se trouve dans une zone de tissus. Dans ce cas, le chirurgien en déduit qu'il n'a pas percé la paroi de la vessie B. Si au contraire le piston se met en mouvement et que la seringue absorbe du liquide, cela signifie que l'extrémité 12 de l'aiguille 10 a pénétré la vessie B. Dans ce dernier cas, il doit arrêter la pénétration et commencer à retirer l'aiguille 10.

Par conséquent, il suffit au chirurgien de, pousser et de tirer alternativement le piston de la seringue 18 pour pratiquer l'anesthésie tout en contrôlant en permanence la position de la pointe 12 de l'aiguille 10.

Pour détecter l'atteinte de la vessie B, le chirurgien peut également injecter un liquide coloré dans la vessie. De cette manière, le contenu de la seringue 18 se colore lorsque celle-ci absorbe du liquide.

Bien entendu, la présente invention n'est pas limitée aux modes de réalisation particuliers qui viennent d'être décrits mais s'étend à toute variante conforme à son esprit.

Par exemple, le dispositif de soutènement sous uréthro-cervical peut comprendre une bande unique dont chaque extrémité est reliée à un élément d'ancrage.

Les éléments d'ancrage peuvent comprendre des stries radiales ou des zones de pliage qui leur permette de se replier plus facilement le long de l'aiguille lorsque celle-ci est enfoncée à travers les tissus.

Les éléments d'ancrage ne sont pas nécessairement circulaires, ils peuvent présenter toute forme permettant de les replier et de les ancrer facilement. Ces éléments peuvent par exemple comprendre des « pétales » souple, par exemple en matériau textile, ces pétales pouvant être repliés le long de l'aiguille ou déployés pour venir en appui sur l'aponévrose pelvienne.

Selon la figure 9, le dispositif d'ancrage 6 comprend une aiguille 10 d'environ un millimètre de diamètre dont l'extrémité 12 destinée à pénétrer à travers les tissus présente une forme générale conique. De même que l'aiguille 2 de la figure 1, cette aiguille 6 présente à proximité de son extrémité conique 12, une surface d'appui 16 en forme de couronne perpendiculaire à sa direction longitudinale. Un élément d'ancrage 29 a été installé emmanché sur l'aiguille 10, en appui sur la surface d'appui 16. Cet élément d'ancrage 9 comprend un embout rigide 26 en polypropylène de forme générale conique présentant un plus grand diamètre d'environ 5 millimètre. Deux portions de bande 7 et 8 formant des ailettes ainsi que l'extrémité d'une bande de soutènement 36 en polypropylène sont fixées sur la base évasée de l'embout 26, par exemple par collage ou soudure. L'embout 26 et les deux ailettes 7 et 8 constituent l'élément d'ancrage 29. Les deux portions de bandes 7 et 8 formant les ailettes sont aptes à être rabattues le long de l'aiguille 10 lorsque celle-ci est enfoncée à travers des tissus biologiques et déployées lorsque l'embout 26 a franchi ces tissus.

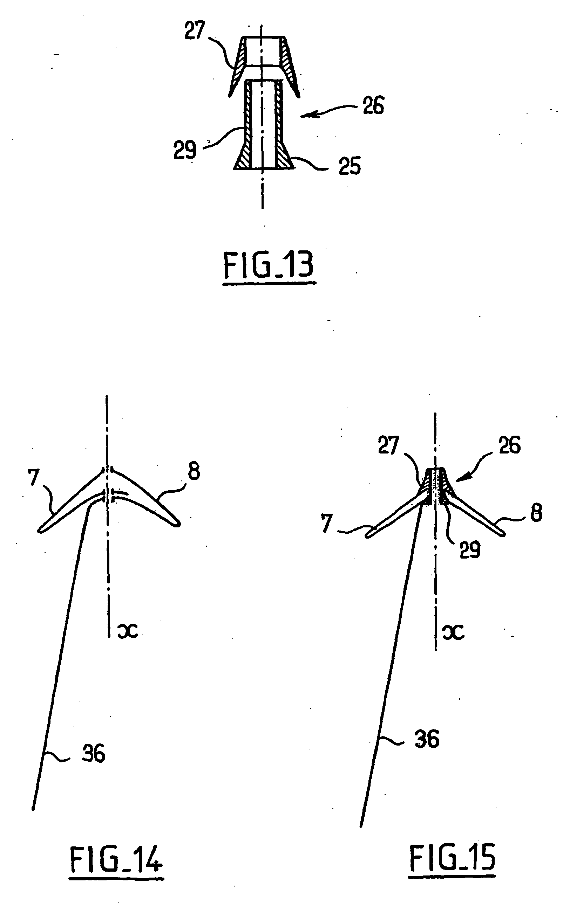

L'élément d'ancrage peut être formé comme représenté sur la figure 13 d'un embout 26 composé d'une première pièce conique 27 dans laquelle vient s'encastrer une deuxième pièce tubulaire 29 complémentaire. La pièce tubulaire 29 présente une portion évasée 25 dans sa partie inférieure. Comme représenté sur la figure 14, l'extrémité d'une bandelette 36 est replié en trois pour former deux ailettes 7 et 8. Les trois épaisseurs de bandelettes repliées sont thermopercées ensemble selon un axe x. La bandelette 36 et les ailettes 7 et 8 ainsi formées sont ensuite positionnées entres les deux pièces 27 et 29. La pièce 29 est introduite dans l'orifice thermopercé traversant les épaisseurs de bandelettes, les bandelettes venant en appui sur la portion évasée 25 de la pièce 29. La pièce 29 soutenant les bandelettes est ensuite encastrée dans la pièce 27. Comme représenté sur la figure 15, la bandelette 36 et les ailettes 7 et 8 sont maintenues dans l'embout 26, prises en sandwich entre les deux pièces 27 et 29. Une aiguille 10 utilisée pour la mise en place du dispositif de soutènement pourra ensuite être introduite dans l'embout 26 suivant l'axe x.

On peut de la même façon concevoir un élément d'ancrage présentant plus de deux portions de bandes réparties en pétale autour de l'embout.

L'élément d'ancrage peut également être constitué des bandelettes et des portions de bandes positionnées en pétale et fusionnées ensemble en position. Dans ce cas, les bandelettes et les portions seront de préférence formées d'un treillis de mèches en matériau thermoplastique. Ce type d'élément d'ancrage présente l'avantage de ne pas nécessiter d'embout rigide de maintient des bandelettes entre elles. On souhaite en effet introduire le moins d'élément dur possible dans la région du vagin qui est une région sensible.

En outre, un autre mode d'implantation d'un dispositif de soutènement sous uréthro-cervical utilise un trocart chirurgical. Un tel trocart est représenté à la figure 10. Le trocart 100 comprend une tige métallique 104 (ou mandrin) cylindrique montée sur un manche 108, terminée par une pointe triangulaire 106 et glissant dans une canule 102 qui n'en laisse dépasser que la pointe 106. Selon cet autre mode d'implantation, le trocart 100 est introduit au niveau du vagin V à proximité de l'urètre U dans la direction D jusqu'à l'aponévrose pelvienne. Puis, la tige métallique 104 est retirée de la canule 102 tandis que cette dernière reste en place dans le corps de la patiente. La canule 102 forme alors un tube de guidage dans lequel peut être introduite l'aiguille 10 supportant l'élément d'ancrage. De la même manière que dans les étapes des figures 4 et 5, l'aiguille 10 traverse l'aponévrose pelvienne A. A cet instant, le chirurgien peut sentir qu'il vient de traverser une partie de tissus moins tendre. Il cesse alors de pousser l'aiguille.

L'aiguille 10 est ensuite retirée de la canule 102, ce qui entraîne le déploiement de l'élément d'ancrage 22, 24 ou 29 dont les bords ou les ailettes 7 et 8 viennent en appui sur la surface de l'aponévrose pelvienne A. Lorsque le chirurgien retire l'aiguille 10, l'élément d'ancrage se déploie et s'appuie sur la surface de l'aponévrose A, ce qui l'empêche d'être entraîné par l'aiguille. L'élément d'ancrage 22, 24 ou 29 se désolidarise alors de l'aiguille 10 et reste en place sur l'aponévrose A. La bandelette 32, 34 ou 36 fixée par son extrémité à celui-ci est ainsi maintenue en place. Enfin, la canule 102 est retirée du corps de la patiente, laissant en place la bandelette.

On recommence la même opération avec un deuxième dispositif d'ancrage supportant une bandelette. Le trocart 100 est à nouveau introduit à travers la paroi du vagin V dans la direction du plexus veineux P, mais de l'autre côté de l'urètre U par rapport à la première bande déjà en place.

Puis, de la même manière que dans le premier mode d'implantation précédemment décrit, le chirurgien relie les deux extrémités des bandelettes et règle la longueur de bandelette entre les deux dispositifs d'ancrage.

Ce deuxième mode de mise en place d'un dispositif de soutènement permet avantageusement de maintenir l'élément d'ancrage 22, 24 ou 29 dans sa position repliée lors de son introduction dans la canule 102. L'élément d'ancrage 22, 24 ou 29 ne peut alors se déployer qu'en sortie de canule.

En outre, l'aiguille 10 peut avantageusement être utilisé pour pratiquer une hydrodissection ou une aérodissection avant l'implantation du dispositif de soutènement, c'est à dire injecter un gaz ou un liquide au sein des tissus pour préparer un volume qui recevra l'élément d'ancrage. Le volume de liquide ou de gaz ainsi créé est plus propice au déploiement de l'élément d'ancrage.

Enfin, les éléments nécessaire à l'implantation d'un soutènement sous uréthro-cervical conforme à l'invention peuvent avantageusement être proposés au chirugien sous la forme d'un « kit » ou d'un ensemble chirurgical comprenant la totalité ou une partie des éléments suivants : le dispositif de soutènement sous uréthro-cervical incluant les bandelettes éventuellement entourées d'une gaine et deux élément d'ancrage, la ou les aiguille(s) d'ancrage, un élément de fixation des bandelettes entre elles, une pince permettant de positionner l'élément de fixation, ainsi qu'un trocart.

## Revendications

1. Dispositif de soutènement sous uréthro-cervical comprenant au moins une bandelette (32 ; 34; 36) et deux éléments d'ancrage (22 ; 24 ; 29), chaque, élément d'ancrage étant relié à une extrémité de bandelette (32 ; 34 ; 36), **caractérisé en ce que** chaque élément d'ancrage (22 ; 24; 26) est un élément souple apte à être installé emmanché sur une extrémité d'une aiguille (10) et apte à être rabattu le long de l'aiguille (10) lorsque l'aiguille (10) traverse des tissus biologiques (A) et déployé lorsqu'il a franchi ces tissus pour venir en appui sur les tissus, et étant également apte à se désolidariser de l'aiguille (10) lorsque l'aiguille est retirée des tissus.

2. Dispositif selon la revendication 1, **caractérisé en ce que** chaque élément d'ancrage (22, 24) est une rondelle souple en silicone renforcée avec du polypropylène.

3. Dispositif selon l'une des revendications 1 ou 2, **caractérisé en ce que** chaque élément d'ancrage (22, 24) est une rondelle souple présentant une forme de cône dont la concavité est destinée à être dirigée vers une extrémité (14) de manipulation de l'aiguille (10) opposée à la pointe de l'aiguille.

4. Dispositif selon l'une des revendications 1 à 3, **caractérisé en ce que** chaque élément d'ancrage (22, 24) comprend des stries radiales ou des zones de pliage permettant de replier l'élément d'ancrage le long de l'aiguille (10) lorsque l'aiguille est enfoncée à travers les tissus.

5. Dispositif selon la revendication 1, **caractérisé en ce que** chaque élément d'ancrage (29) comprend un embout (26) de forme générale conique et au moins deux portions de bandelettes (7, 8) formant des ailettes.

6. Dispositif selon la revendication 5, **caractérisé en ce que** l'embout (26) comprend deux pièces rigides (27, 29) aptes à venir s'encastrer l'une dans l'autre et entre lesquelles sont maintenues les bandelettes (36) ou portions de bandelettes (7, 8).

7. Dispositif selon la revendications 5, **caractérisé en ce que** chaque élément d'ancrage est constitué de portions bandelettes rassemblées et fusionnées ensemble en position.

8. Dispositif selon l'une des revendications 1 à 7, **caractérisé en ce qu'**il comporte deux bandelettes (32, 34, 36), chacune des bandelettes ayant une extrémité reliée à l'un des éléments d'ancrage (22 ; 24 ; 29).

9. Dispositif selon la revendication 8, **caractérisé en ce qu'**il comprend des moyens de connexion (42 ; 44) pour les relier les deux bandelettes (32, 34, 36) l'une à l'autre.

10. Dispositif selon l'une des revendications 1 à 9, **caractérisé en ce que** chaque bandelette (32 ; 34 ; 36) est en polypropylène.

11. Ensemble chirurgical (2, 4, 6) pour l'implantation d'un soutènement sous-uréthro cervical, comprenant un dispositif selon l'une des revendications 1 à 10, et une aiguille (10) apte à supporter un élément d'ancrage (22 ; 24 ; 26).

12. Ensemble selon la revendication 11, **caractérisé en ce que** l'aiguille (10) présente à proximité de son extrémité traversante une surface d'appui (16) supportant l'élément d'ancrage (22, 24, 29).

13. Ensemble selon l'une des revendications 11 ou 12, **caractérisé en ce que** l'aiguille (10) est creuse et forme un canal débouchant à ses deux extrémités (12, 14) et **en ce que** l'extrémité (14) de l'aiguille (10) ne supportant pas l'élément d'ancrage (22 ; 24) est apte à être connectée à des moyens d'injection/aspiration (18).

14. Ensemble selon l'une des revendications 11 à 13, **caractérisé en ce que** la ou les bandelettes (32, 34, 36) sont entourées d'une gaine de protection.

15. Ensemble chirurgical pour l'implantation d'un soutènement sous uréthto-cervical selon l'une des revendications 11 à 14, comprenant une pince (50 ; 51) apte à disposer par serrage les moyens de connexion sur les bandelettes (32, 34, 36). apte à recevoir une aiguille (10) supportant un élément d'ancrage (22, 24, 29).

16. Ensemble chirurgical selon la revendication 15, **caractérisé en ce qu'**il comprend en outre un trocart (100) présentant une canule (102)

## Claims

1. Sub-urethro-cervical support device comprising at least one striplet (32; 34; 36) and two anchor elements (22; 24; 29), each anchor element being connected to an end of a striplet (32; 34; 36), **characterised in that** each anchor element (22; 24; 26) is a flexible element capable of being fitted by pressing on one end of a needle (10) and capable of being turned down over the length of the needle (10) while the needle (10) is passing through biological tissues (A) and deployed when it has passed through these tissues to rest on the tissues, and also capable of being detached from the needle (10) when the needle is removed from the tissues.

2. Device according to claim 1, **characterised in that** each anchor element (22, 24) is a flexible washer made of silicone reinforced with polypropylene.

3. Device according to any of claims 1 to 2, **characterised in that** each anchor element (22, 24) is a flexible washer having a conical shape wherein the concavity is intended to be directed towards an end (14) for handling the needle (10) opposite the tip of the needle.

4. Device according to any of claims 1 to 3, **characterised in that** each anchor element (22, 24) comprises radial striations or folding zones so that the anchor element can be more easily folded along the needle (10) when the needle is pushed through the tissues.

5. Device according to claim 1, **characterised in that that** each anchor element (29) has a conically shaped end piece (26), and at least two portions of striplets (7, 8) forming winglets.

6. Device according to claim 5, **characterised in that** the end piece (26) comprises two rigid parts (27, 29) that can be inserted one into the other, between which striplets (36) or portions of striplets (7, 8) are held.

7. Device according to claim 5, **characterised in that** the anchor elements comprise striplet portions arranged and fused together in position.

8. Device according to any of claims 1 to 7, **characterised in that** it comprises two striplets (32, 34, 36), each of which has one end connected to one of the anchor elements (22; 24; 29).

9. Device according to claim 8, **characterised in that** it comprises connection means (42; 44) to connect the two striplets (32, 34, 36) together.

10. Device according to any of claims 1 to 9, **characterised in that** each striplet (32; 34; 36) is made of polypropylene.

11. Surgical assembly (2, 4, 6) for implantation of a sub-urethro-cervical support, comprising a device according to any of claims 1 to 10, and a needle (10) capable of supporting an anchor element (22; 24; 26).

12. Assembly according to claim 11, **characterised in that** the needle (10) has a bearing surface (16) supporting the anchor element (22, 24, 29), close to its penetrating end.

13. Assembly according to any of claims 11 or 12, **characterised in that** the needle (10) is hollow and forms a duct opening up at its two ends (12, 14) and **in that** the end (14) of the needle (10) not supporting the anchor element (22; 24), can be connected to injection/suction means (18).

14. Assembly according to any of claims 11 to 13, **characterised in that** the striplet(s) (32, 34, 36) is (are) surrounded by a protective sheath.

15. Surgical assembly for implantation of a sub-urethro-cervical support according to any of claims 11 to 14, comprising a clamp (50; 51), capable of tightening the connection means on the striplets (32, 34, 36).

16. Surgical assembly according to claim 15, **characterised in that** it also comprises a trocar (100) that comprises a canula (102) capable of lousing the needle (10) supporting an anchor element (22, 24, 29).

## Patentansprüche

1. Suburethrale-zervikale Stützvorrichtung, welche wenigstens ein Bändchen (32; 34; 36) und zwei Verankerungselemente (22; 24; 29) umfasst, wobei jedes Verankerungselement mit einem Bändchenende (32; 34; 36) verbunden ist, **dadurch gekennzeichnet, dass** jedes Verankerungselement (22; 24; 26) ein nachgiebiges Element ist, das ausgelegt ist, aufgesteckt auf einem Ende einer Nadel (10) eingerichtet zu werden und ausgelegt ist, entlang der Nadel (10) umgelegt zu werden, wenn die Nadel (10) biologische Gewebe (A) durchquert, und ausgebreitet zu werden, wenn es diese Gewebe überwunden hat, um auf den Geweben anzuliegen, und auch ausgelegt ist, sich von der Nadel (10) zu trennen, wenn die Nadel aus den Geweben zurückgezogen wird.

2. Vorrichtung gemäß Anspruch 1, **dadurch gekennzeichnet, dass** jedes Verankerungselement (22, 24) eine nachgiebige Scheibe aus mit Polypropylen verstärktem Silikon ist.

3. Vorrichtung gemäß einem der Ansprüche 1 oder 2, **dadurch gekennzeichnet, dass** jedes Verankerungselement (22, 24) eine Kegelform aufweist, deren Konkavität dazu bestimmt ist, in Richtung eines Bedienendes (14) der Nadel (10) gegenüber der Spitze der Nadel gerichtet zu sein.

4. Vorrichtung gemäß einem der Ansprüche 1 bis 3, **dadurch gekennzeichnet, dass** jedes Verankerungselement (22, 24) radiale Streifen oder Faltungsbereiche umfasst, die erlauben, das Verankerungselement entlang der Nadel (10) zusammenzufalten, wenn die Nadel durch die Gewebe gestochen wird.

5. Vorrichtung gemäß Anspruch 1, **dadurch gekennzeichnet, dass** jedes Verankerungselement (29) ein Ansatzstück (26) von allgemeiner Kegelform und wenigstens zwei Teile von flügelbildenden Bändchen (7,8) umfasst.

6. Vorrichtung gemäß Anspruch 5, **dadurch gekennzeichnet, dass** das Ansatzstück (26) zwei starre Stücke (27, 29) umfasst, die ausgelegt sind, sich ineinander zu fügen und zwischen denen die Bändchen (36) oder Teile von Bändchen (7,8) gehalten werden.

7. Vorrichtung gemäß Anspruch 5, **dadurch gekennzeichnet, dass** jedes Verankerungselement aus Teilen von zusammengestellten und an Ort und Stelle zusammengeschlossenen Bändchen gebildet ist.

8. Vorrichtung gemäß einem der Ansprüche 1 bis 7, **dadurch gekennzeichnet, dass** sie zwei Bändchen (32, 34, 36) umfasst, wobei jedes der Bändchen ein Ende hat, das mit dem einem der Verankerungselemente (22; 24; 29) verbunden ist.

9. Vorrichtung gemäß Anspruch 8, **dadurch gekennzeichnet, dass** sie Verbindungsmittel (42; 44) umfasst, um die beiden Bändchen (32, 34, 36) miteinander zu verbinden.

10. Vorrichtung gemäß einem der Ansprüche 1 bis 9, **dadurch gekennzeichnet, dass** jedes Bändchen (32; 34; 36) aus Polypropylen ist.

11. Chirurgische Gesamtheit (2, 4, 6) zur Implantierung einer suburethralen-zervikalen Stütze, welche eine Vorrichtung gemäß einem der Ansprüche 1 bis 10 umfasst und eine Nadel (10), die ausgelegt ist, ein Verankerungselement (22; 24; 26) zu stützen.

12. Gesamtheit gemäß Anspruch 11, **dadurch gekennzeichnet, dass** die Nadel (10) nahe ihres durchquerenden Endes eine Auflagefläche (16) aufweist, die das Verankerungselement (22, 24, 29) stützt.

13. Gesamtheit gemäß einem der Ansprüche 11 oder 12, **dadurch gekennzeichnet, dass** die Nadel (10) hohl ist und einen an ihren Enden (12, 14) einmündenden Kanal bildet, und dass das Ende (14) der Nadel (10), das nicht das Verankerungselement (22; 24) stützt, ausgelegt ist, mit Injektions-/Saugmitteln (18) verbunden zu werden.

14. Gesamtheit gemäß einem der Ansprüche 11 bis 13, **dadurch gekennzeichnet, dass** das oder die Bändchen (32, 34, 36) mit einer Schutzummantelung umgeben sind.

15. Chirurgische Gesamtheit zur Implantierung einer suburethralen-zervikalen Stütze gemäß einem der Ansprüche 11 bis 14, sowie eine Zange (50; 51), die ausgelegt ist, die Verbindungsmittel auf den Bändchen (32, 34, 36) durch Klemmen anzuordnen.

16. Chirurgische Gesamtheit gemäß Anspruch 15, **dadurch gekennzeichnet, dass** sie außerdem einen Trokar (100) umfasst, der eine Kanüle (102) aufweist, die ausgelegt ist, eine Nadel (10) aufzunehmen, die ein Verankerungselement (22, 24, 29) stützt.
